Europäisches Patentamt

European Patent Office

Office européen des brevets

(19)

(11) **EP 0 969 781 B1**

(12) **EUROPÄISCHE PATENTSCHRIFT**

(45) Veröffentlichungstag und Bekanntmachung des Hinweises auf die Patenterteilung:
**18.12.2002 Patentblatt 2002/51**

(21) Anmeldenummer: **98916955.2**

(22) Anmeldetag: **16.03.1998**

(51) Int Cl.⁷: $A61F\ 2/36$, $A61F\ 2/32$

(86) Internationale Anmeldenummer:
**PCT/EP98/01503**

(87) Internationale Veröffentlichungsnummer:
**WO 98/041172 (24.09.1998 Gazette 1998/38)**

(54) **KÜNSTLICHER GELENKKOPF FÜR DAS MENSCHLICHE HÜFTGELENK**

ARTIFICIAL CONDYLE FOR THE HUMAN HIP JOINT

CONDYLE ARTIFICIEL POUR ARTICULATION DE LA HANCHE HUMAINE

(84) Benannte Vertragsstaaten:
**CH DE FR GB LI**

(30) Priorität: **15.03.1997 DE 19710934**

(43) Veröffentlichungstag der Anmeldung:
**12.01.2000 Patentblatt 2000/02**

(73) Patentinhaber: **HJS Gelenk System GmbH
81925 München (DE)**

(72) Erfinder:
• **KUBEIN-MEESENBURG, Dietmar
D-37547 Kreiensen (DE)**
• **NÄGERL, Hans
D-37130 Gleichen (DE)**

(74) Vertreter: **Braun, Dieter, Dipl.-Ing. et al
Hagemann, Braun & Held
Patentanwälte,
Hildesheimer Strasse 133
30173 Hannover (DE)**

(56) Entgegenhaltungen:
**EP-A- 0 590 241        WO-A-96/04867
DE-A- 3 908 958        US-A- 5 556 432**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft einen künstlichen Gelenkkopf zur Verwendung in einem menschlichen Hüftgelenk mit einer sphärisch ausgebildeten im Querschnitt eine konkave Schnittkontur aufweisende Gelenkschale mit einem Krümmungsradius $R_1$ und einem Krümmungsmittelpunkt $M_1$. Dieser Gelenkkopf besitzt zumindest in seinem in der Gelenkschale artikulierenden Bereich eine derart sphärisch geformte Gelenkfläche, daß im eingesetzten Zustand in der Hüftschale in einer durch den Mittelpunkt $M_1$ der Hüftschale verlaufenden Längsebene ein Radius $R_{K1} = R_1$ ($R_1$ gleich Radius der Hüftschale) mit einem Mittelpunkt $M_{K1}$ ausgebildet ist, der mit dem Mittelpunkt $M_1$ zusammenfällt, und in einer zu der Längsebene senkrechten Querebene durch den Mittelpunkt $M_{K1}$ ein Radius $R_{K2} < R_{K1}$ mit dem Mittelpunkt $M_{K2}$ vorhanden ist, und in der Querebene eine stabile dimere Gelenkkette ausgebildet ist, bei der die Gelenkachsenbahn der Mittelpunkte $M_{K1}$ und $M_{K2}$ einen Radius $R = R_{K1} - R_{K2}$ besitzt, wobei R eine positive Größe ist.

Aus der DE-PS 39 08 958 ist ein künstliches Gelenk zum Ersatz des menschlichen Hüftgelenks bekannt, das aus mindestens zwei künstlichen Gelenkteilen mit zueinander sich bewegenden sphärischen Funktionsflächen besteht, wobei die Krümmungsverhältnisse der eine kreisförmige Schnittkontur aufweisenden Funktionsflächen zueinander konvex/konkav derart ausgebildet sind, daß ihre Rotationszentren $M_1$ und $M_2$ innerhalb des Gelenkteils mit der konvexen Funktionsfläche liegen und zwischen den beiden Funktionsflächen ein Druckverteilungskörper als drittes künstliches Gelenkteil angeordnet ist. Die Ausgestaltung dieses künstlichen Gelenks ist derart, daß eine stabile Konfiguration geschaffen wird, wobei eine Druckkraft längs der Verbindungslinie der beiden Krümmungsmittelpunkte zu einem stabilen Lagezustand führt. Der Druckverteilungskörper richtet sich zwischen den beiden Gelenkteilen derart aus, daß er nicht seitlich ausscheren kann. Dieses Gelenk ist wie eine Gelenkkette mit zwei Gelenkachsen aufgebaut, d. h. es handelt sich um eine sogenannte dimere Gelenkkette, wobei aufgrund der speziellen Ausgestaltung eine druckstabile dimere Gelenkkette gegeben ist. Dieses bekannte künstliche Hüftgelenk läßt eine Bewegung in fünf Freiheitsgraden für einen daran gekoppelten Körper oder ein Gliedmaß zu. Dies wird dadurch erreicht, daß der Mittelpunkt des Gelenkkopfes und der Mittelpunkt der Gelenkpfanne nicht zusammenfallen und diese durch ihren konstanten Abstand unter kraftschluß die kraftübertragende dimere Kette bilden. Durch die Verwendung des zusätzlichen Druckverteilungskörpers sind die Herstellungskosten dieses Gelenks erhöht gegenüber künstlichen Hüftgelenken aus nur zwei Gelenkteilen. Darüber hinaus ist das Einsetzen dieses aus drei Teilen bestehenden Hüftgelenks im menschlichen Körper aufwendiger als bei einem zweiteiligen Hüftgelenk.

Aus der EP-A-0 590 241 ist ein künstliches Schultergelenk mit einem Gelenkkopf bekannt, der die Merkmale des Oberbegriffs von Anspruch 1 aufweist.

Der vorliegenden Erfindung liegt die Aufgabe zugrunde, eine Endoprothese für den Hüftkopf eines menschlichen Hüftgelenks zu schaffen, bei der auf einen zusätzlichen Druckverteilungskörper verzichtet werden kann, jedoch in der Hauptfunktionsebene eine druckstabile überschlagene dimere Kette realisiert werden kann, die bei Schwenken der Hauptfunktionsebene immer gleichgroß ist.

Erfindungsgemäß wird dies dadurch erreicht, daß bei einem gattungsgemäßen Gelenkkopf der Radius $R_{K1}$ über den gesamten Umfang konstant ist.

Erfindungsgemäß wird dieser Gelenkkopf derart in die Gelenkschale unter Bildung eines künstlichen Hüftgelenks oder eines teilweise künstlichen Hüftgelenks eingesetzt, daß die Querebene des Gelenkkopfes mit der Hauptfunktionsebene des Gelenks zusammenfällt. Die Längsebene des erfindungsgemäßen Gelenkkopfes steht hierbei senkrecht auf der Hauptfunktionsebene. Die Radiusdiskrepanz zwischen dem Radius der sphärischen Pfanne mit der im Querschnitt kreisförmigen konkaven Funktionsfläche und dem kleineren Radius $R_{K2}$ gibt dann in der Hauptfunktionsebene eine überschlagene stabile dimere Gelenkkette vor, wobei die Radiusdiskrepanz erfindungsgemäß vorteilhafterweise zwischen 0,5 bis 10 mm betragen kann. Aufgrund der erfindungsgemäßen Ausgestaltung des Gelenkkopfes kann in allen Funktionsstellungen des Gelenkkopfes in der Gelenkschale ein linienförmiger Kontakt zwischen Gelenkkopf und Gelenkschale erreicht werden. Die erfindungsgemäße Konstruktion erlaubt es, daß bei Gliedmaßen, die mit einem mit dem erfindungsgemäßen Gelenkkopf ausgestatteten Gelenk verbunden sind, die Hauptfunktionsebene geschwenkt werden kann und dabei garantiert ist, daß in dieser immer eine gleichgroße dimere Kette vorhanden ist. In der Senkrechten zur Hauptfunktionsebene ist aufgrund der erfindungsgemäßen Ausgestaltung keine dimere Kette oder nur eine minimale dimere Kette (bis maximal 1/10 der in der Hauptfunktionsebene bestehenden dimeren Kette) in der Grundstellung vorhanden.

Die vorliegende Erfindung erstreckt sich nicht nur auf einen künstlichen Gelenkkopf, sondern auch auf ein künstliches Gelenk aus dem erfindungsgemäßen vorgeschriebenen Gelenkkopf und einer künstlichen Gelenkschale mit einer kreisförmigen Schnittkontur mit dem Radius $R_1$ und dem Mittelpunkt $M_1$.

Weitere vorteilhafte Ausführungen sind in den Unteransprüchen enthalten.

Anhand des in den beiliegenden Zeichnungen dargestellten Ausführungsbeispiels wird die Erfindung näher erläutert.

Es zeigen:

Fig. 1        eine Ansicht von oben auf eine Gelenkschale eines erfindungsgemäßen Gelenks mit eingesetztem Gelenkkopf in einem im wesentlichen horizontalen Schnittdarstellung,

Fig. 2　einen Schnitt entlang der Schnittlinie II-II in Fig. 1,

Fig. 3　einen Schnitt entlang der Schnittlinie III-III in Fig. 1 und

Fig. 4 und 5　Schnitte entlang der Schnittlinie III-III in Fig. 1 in von Fig. 3 abweichenden Stellungen des erfindungsgemäßen Gelenkkopfes.

Fig. 6　eine Seitenansicht, zum Teil geschnitten, eines erfindungsgemäßen Gelenks,

Fig. 7　eine Einzelheit gemäß X in Fig. 6,

Fig. 8　einen Schnitt in der X-Z-Ebene in Fig. 7 und

Fig. 9　einen Schnitt entlang der der Y-Z-Ebene in Fig. 7.

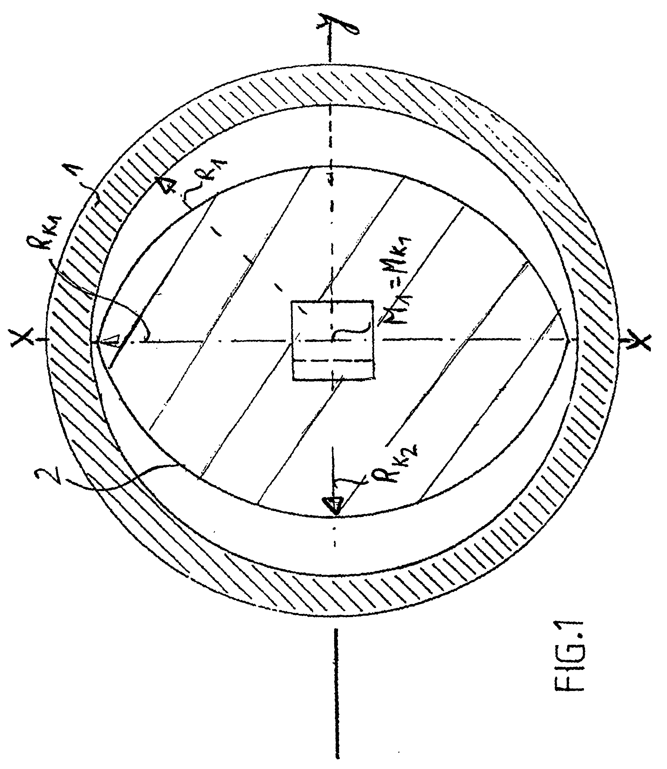

In den Figuren 1 bis 9 sind gleiche Teile mit denselben Bezugsziffern versehen.

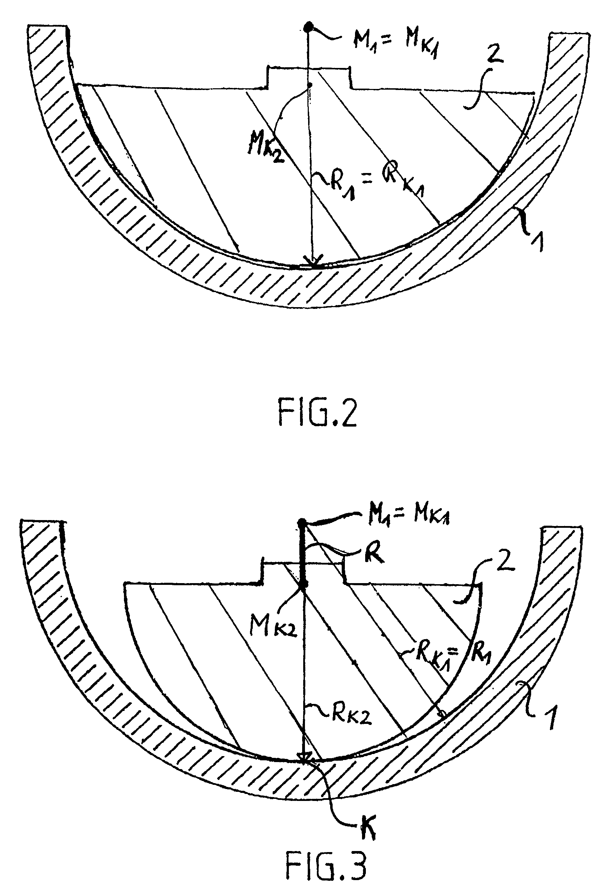

Wie aus Fig. 1 zu erkennen ist, besteht ein erfindungsgemäßes Gelenk aus einer Gelenkschale 1 und einem Gelenkkopf 2. Diese beiden künstlichen Gelenkteile können aus den bekannten Materialien künstlicher Gelenke hergestellt sein. Die Gelenkschale 1 hat eine sphärische Funktionsfläche mit einer kreisförmigen, konkaven Schnittkontur mit dem Radius $R_1$ und dem Mittelpunkt $M_1$. In diese Gelenkschale 1 ist der Gelenkkopf 2 beweglich eingesetzt. Der Gelenkkopf 2 besitzt eine sphärische konvexe Funktionsfläche, zumindest in dem Bereich, mit dem er innerhalb der Gelenkschale 1 artikuliert. Diese sphärische Funktionsfläche ist nun derart ausgestaltet, daß im in der Gelenkschale 1 eingesetzten Zustand der Gelenkkopf 2 in einer durch den Mittelpunkt $M_1$ verlaufenden Längsebene X-X einen Radius $R_{K1}$ besitzt, der gleich, zumindest aber nahezu gleich $R_1$ der Gelenkschale ist, wobei dem Radius $R_{K1}$ ein Mittelpunkt $M_{K1}$ zugeordnet ist, der mit dem Mittelpunkt $M_1$ der Gelenkschale zusammenfällt. In einer zur Längsebene X-X senkrechten Querebene Y-Y, die durch den Mittelpunkt $M_{K1}$ verläuft, besitzt die konvexe, sphärische Funktionsfläche des Gelenkkopfes 2 einen Radius $R_{K2}$, der kleiner ist als der Radius $R_{K1}$. Hierdurch wird in der Querebene eine stabile dimere überschlagene Gelenkkette gebildet, die einen Gelenkbahnradius R aufweist, siehe Fig. 3, dabei gilt R = $R_{K1}$ - $R_{K2}$. Die sphärische Funktionsfläche des erfindungsgemäßen Gelenkkopfes 2 ist zwischen den Radien $R_{K1}$ und $R_{K2}$ in der Längsebene bzw. der Querebene ausgestaltet.

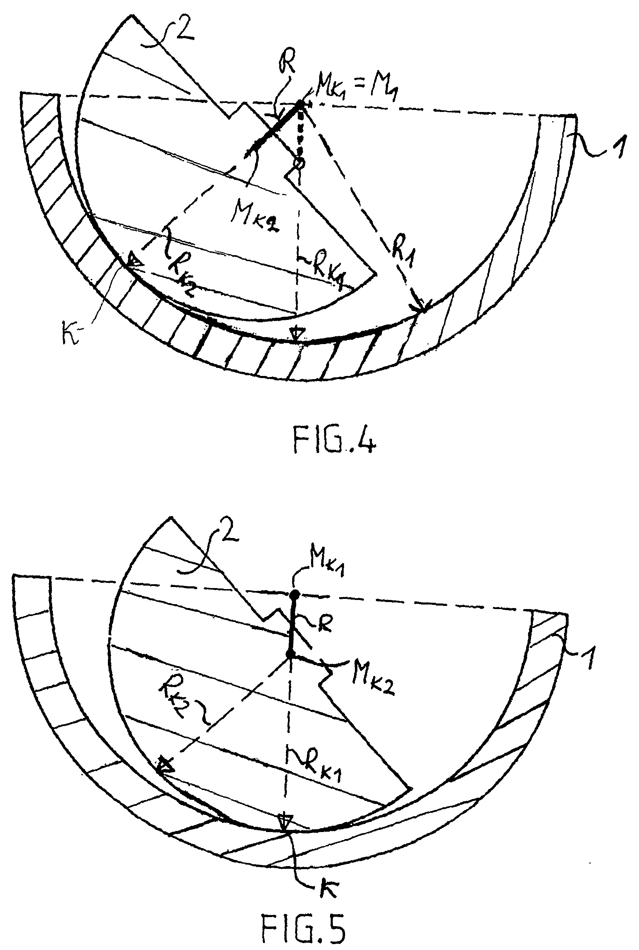

Die Größendifferenz zwischen den Radien $R_{K1}$ und $R_{K2}$ des erfindungsgemäßen Gelenkkopfes beträgt vorzugsweise 1 bis 10 mm. Der erfindungsgemäße Gelenkkopf 2 ist jeweils derart in die Gelenkschale 1 einzusetzen, daß seine Querebene Y-Y mit der Hauptfunktionsebene des eingesetzten künstlichen Gelenks bzw. des mit dem erfindungsgemäßen Gelenkkopfs 2 zu bildenden Hüftgelenks zusammenfällt. Wie sich aus den Figuren 4 und 5 ersehen läßt, ist in jeder Bewegungsstellung des Gelenkkopfes 2 in der Gelenkschale 1 zwischen diesen beiden ein linienförmiger Kontakt entlang einer Kontaktfläche durch den Kontaktpunkt K vorhanden. In der Fig. 4 ist dabei eine Gleitbewegung zwischen dem Gelenkkopf 2 und der Gelenkschale 1 um den Mittelpunkt $M_1$ dargestellt und in Fig. 5 ist eine Gleitbewegung des Gelenkkopfes 2 in der Gelenkschale 1 um den Mittelpunkt $M_2$ dargestellt. Beide Bewegungen gemäß den Figuren 4 und 5 überlagern sich bei der Bewegung eines Gliedmaßes, das mit dem erfindungsgemäßen Gelenk verbunden ist. In der Längsebene, wie in Fig. 2 dargestellt, erfolgt eine Drehbewegung des Gelenkkopfes 2 in der Gelenkschale 1 um den Mittelpunkt $M_1$.

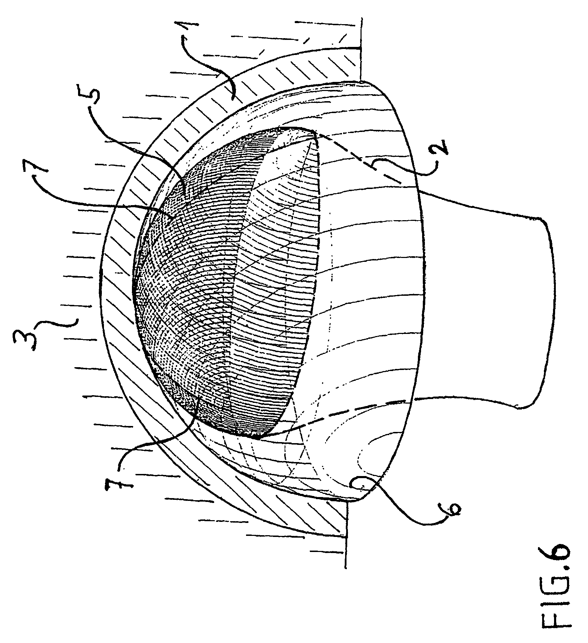

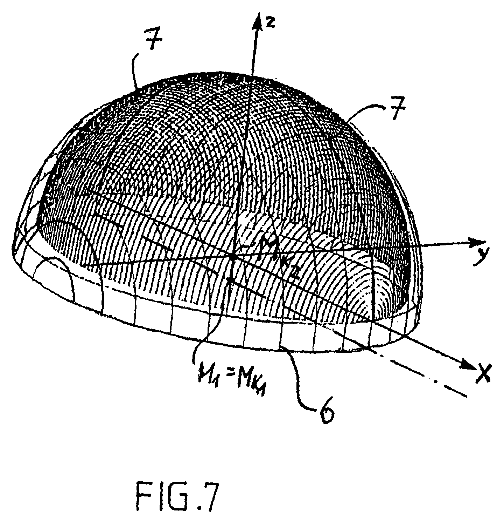

In Fig. 6 ist ein erfindungsgemäßes Gelenk dargestellt, wobei der künstliche Gelenkkopf 2 in die künstliche Gelenkschale 1 eingesetzt ist. Die künstliche Gelenkschale 1 ist innerhalb eines natürlichen Hüftknochens 3 befestigt. Zwischen dem Gelenkkopf 2 und der Gelenkschale 1 bildet sich an deren Artikulationsflächen 5 und 6 ein linienförmiger Kontakt entlang einer Kontaktlinie 7. Dieser linienförmiger Kontakt bleibt in jeder Bewegungsstellung erhalten. Aus den Fig. 7 bis 9 ergeben sich die geometrischen Angaben bzw. Parameter, mit denen die Gestalt der sphärischen Funktionsfläche (Artikulationsfläche 5) des Gelenkkopfes 2 bestimmt werden kann.

In Fig. 7 wird durch die Kontaktlinie 7 die Z-X-Ebene eines Bezugs-Koordinatensystems definiert. Die Y-Z-Ebene dieses Koordinatensystems stellt die Hauptfunktionsebene des erfindungsgemäßen Gelenks dar. Sie ist die Symmetrieebene der zweizähligen Symmetrie des Gelenkkopfes 2, der einen etwa tonnenförmigen Körper bildet. Es gilt:

$$X = R_1 \cdot \cos\alpha$$

$$Y = [R_1 \cdot \sin\alpha - (R_1 - R_2)] \cdot \cos\beta$$

$$Z = [R_1 \cdot \sin\alpha - (R_1 - R_2)] \cdot \sin\beta$$

Hierbei gelten folgende Wertbeschränkungen

$$0 \leq \beta \leq \Pi/2$$

$$\arcsin [(R_1 - R_2)/R_1] \leq \alpha \leq \Pi/2.$$

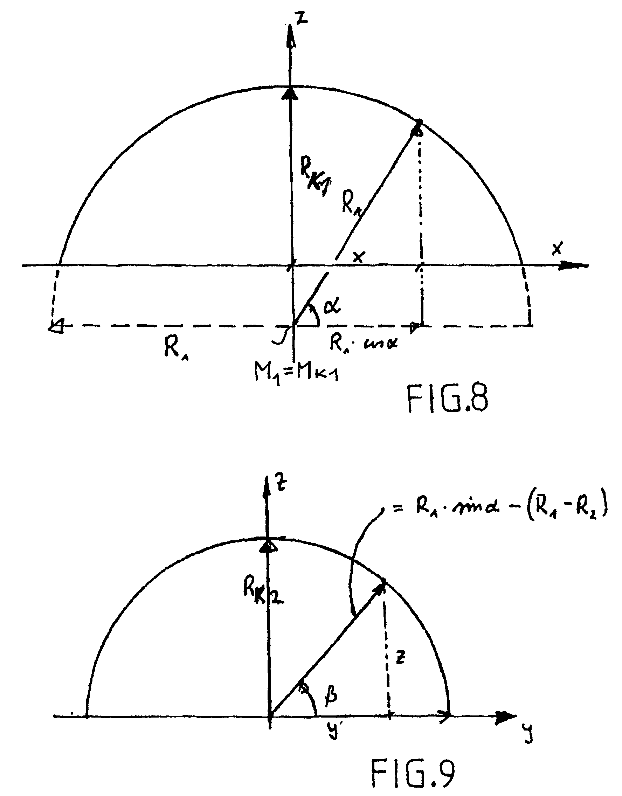

Die Definition der Winkel $\alpha$ und $\beta$ ergibt sich aus den Fig. 8 und 9.

Gegenüber bekannten Hüftgelenk-Endoprothesen, die als reine Kugelgelenke ausgebildet sind, zeichnet sich das erfindungsgemäße Gelenk durch eine in der Hauptfunktionsebene bestehende druckstabile Anordnung aus, wobei punktförmige Berührungskontakte zwischen den Gelenkkörpern vermieden werden, und vielmehr linienförmige Kontakte sichergestellt sind, so daß die Belastung der einzelnen Gelenkkörper wesentlich reduziert wird.

**Patentansprüche**

1. Künstlicher Gelenkkopf (2) zur Verwendung in einem menschlichen Hüftgelenk mit einer sphärisch ausgebildeten im Querschnitt eine kreisförmige, konkave Schnittkontur aufweisenden Gelenkschale (1) mit einem Krümmungsradius $R_1$ und einem Krümmungsmittelpunkt $M_1$, wobei der Gelenkkopf (2) eine zumindest in ihrem in der Gelenkschale (1) artikulierenden Bereich derart sphärisch konvex geformte Funktionsfläche aufweist, die im in der Schale (1) eingesetzten Zustand in einer durch den Mittelpunkt $M_1$ verlaufenden Längsebene X-X einen Radius $R_{K1} = R_1$ mit einem Mittelpunkt $M_{K1}$ aufweist, der mit $M_1$ zusammenfällt, und die in einer zu der Längsachse X-X senkrechten Querebene Y-Y durch den Mittelpunkt $M_{K1}$ einen Radius $R_{K2} < R_{K1}$ mit dem Mittelpunkt $M_{K2}$ aufweist, so daß in der Querebene Y-Y eine stabile dimere Gelenkkette gebildet ist, bei der die Gelenkachsenbahn der Mittelpunkte $M_{K1}$ und $M_{K2}$ einen Gelenkbahnradius $R = R_{K1} - R_{K2}$ aufweist, **dadurch gekennzeichnet, daß** der Radius $R_{K1}$ der Funkstionsfläche des Gelenkkopfes über den gesamten Umfang konstant ist.

2. Gelenkkopf nach Anspruch 1,
**dadurch gekennzeichnet, daß** die Größendifferenz zwischen $R_{K1}$ und $R_{K2}$ 0,5 bis 10 mm beträgt.

3. Künstliches Gelenk zum Ersatz des menschlichen Hüftgelenks, bestehend aus einer künstlichen sphärischen Gelenkschale mit einer konkaven kreisförmigen Schnittkontur mit dem Radius $R_1$ und dem Mittelpunkt $M_1$ sowie einem in der Schale beweglich angeordneten sphärischen Gelenkkopf mit einer konvexen Funktionsfläche,
**dadurch gekennzeichnet, daß** der Gelenkkopf gemäß Anspruch 1 oder 2 ausgebildet ist.

4. Künstliches Gelenk nach Anspruch 3,
**dadurch gekennzeichnet, daß** die Querebene Y-Y mit der Hauptfunktionsebene (Y-Z) des Gelenks zusammenfällt (siehe Fig. 7).

**Claims**

1. Artificial condyle (2) for use in a human hip joint with a spherically formed joint socket (1) comprising a concave sectional contour, which is circular in cross-section, with a radius of curvature $R_1$ and a centre of curvature $M_1$, the condyle (2) comprising an operational face which is spherically convexly shaped, at least in its region articulating in the joint socket (1) and, when inserted in the socket (1), has a radius $R_{K1} = R_1$ in a longitudinal plane X-X extending through the centre $M_1$, with a centre $M_{K1}$ coinciding with $M_1$, and has a radius $R_{K2} < R_{K1}$ with the centre $M_{K2}$ in a transverse plane Y-Y perpendicular to the longitudinal axis X-X through the centre $M_{K1}$ so in the transverse plane Y-Y a stable dimeric joint chain is formed in which the joint axis path of the centres $M_{K1}$ and $M_{K2}$ has a joint path radius $R = R_{K1} - R_{K2}$, **characterised in that** the radius $R_{K1}$ of the operational face of the condyle is constant over the entire circumference.

2. Condyle according to claim 1, **characterised in that** the difference in size between $R_{K1}$ and $R_{K2}$ is 0.5 to 10 mm.

3. Artificial joint for replacement of the human hip joint, consisting of an artificial, spherical joint socket with a concave circular sectional contour with the radius $R_1$ and the centre $M_1$ and a spherical condyle with a convex operational face movably arranged in the socket, **characterised in that** the condyle is designed in accordance with claim 1 or 2.

4. Artificial joint according to claim 3, **characterised in that** the transverse plane Y-Y coincides with the main operational plane (Y-Z) of the folded joint (see Fig. 7).

**Revendications**

1. Condyle artificiel (2) destiné à une articulation de hanche humaine avec une cavité cotyloïde (1) de forme sphérique et présentant en section transversale un contour de coupe circulaire concave, avec un rayon de courbure $R_1$ et un point central de courbure $M_1$, le condyle (2) présentant, du moins dans sa zone s'articulant dans la cavité cotyloïde (1), une surface fonctionnelle de forme sphérique et convexe qui, à l'état inséré dans la cavité (1), présente dans un plan longitudinal X-X traversant le point central $M_1$ un rayon $R_{K1} = R_1$ avec un point central $M_{K1}$ qui coïncide avec $M_1$, et qui présente dans un plan transversal Y-Y perpendiculaire à l'axe longitudinal X-X et traversant le point central $M_{K1}$ un rayon $R_{K2} < R_{K1}$ avec le point central $M_{K2}$, de manière à former dans le plan transversal Y-Y une chaîne d'articulation dimère stable où la ligne axiale d'articulation des points centraux $M_{K1}$ et $M_{K2}$ présente un rayon de ligne d'articulation $R = R_{K1} - R_{K2}$, **caractérisé en ce que** le rayon $R_{K1}$ de la surface fonctionnelle du condyle est constant sur toute la circonférence.

2. Condyle selon la revendication 1, **caractérisé en ce que** la différence de taille entre $R_{K1}$ et $R_{K2}$ est de 0,5 à 10 mm.

3. Articulation artificielle pour remplacer l'articulation de hanche humaine, comprenant une cavité cotyloïde sphérique artificielle ayant un contour de coupe circulaire concave avec le rayon $R_1$ et le point central $M_1$, ainsi qu'un condyle sphérique disposé de façon mobile dans la cavité et présentant une surface fonctionnelle convexe, **caractérisée en ce que** le condyle est configuré selon la revendication 1 ou 2.

4. Articulation artificielle selon la revendication 3, **caractérisée en ce que** le plan transversal Y-Y coïncide avec le plan fonctionnel principal (Y-Z) de l'articulation (voir figure 7).

FIG.1

FIG.2

FIG.3

FIG.4

FIG.5

FIG.6

EP 0 969 781 B1

FIG.7

EP 0 969 781 B1

FIG.8

FIG.9

11